(19) Europäisches Patentamt

European Patent Office

Office européen des brevets

(11) Publication number: **0 354 917 B1**

## EUROPEAN PATENT SPECIFICATION

(45) Date of publication of patent specification: **01.06.94**   (51) Int. Cl.⁵: **A61K 33/02**

(21) Application number: **88903625.7**

(22) Date of filing: **16.03.88**

(86) International application number:
**PCT/US88/00950**

(87) International publication number:
**WO 88/07372 (06.10.88 88/22)**

(54) **METHOD OF PROLONGING CANCEROUS PATIENT SURVIVAL IN HUMANS WITH HYDRAZINE SULFATE.**

(30) Priority: **27.03.87 US 32051**

(43) Date of publication of application:
**21.02.90 Bulletin 90/08**

(45) Publication of the grant of the patent:
**01.06.94 Bulletin 94/22**

(84) Designated Contracting States:
**AT BE CH DE FR GB IT LI LU NL SE**

(56) References cited:
**US-A- 4 110 437**

**NUTRITION & CANCER, vol. 9, nos. 2-3, 1987,
Lawrence Erlbaum Associates, Inc.; J.GOLD,
pp. 55-66.**

**JOURNAL OF PRACTICAL NURSING, vol. 3,
no. 1, March 1988; N.M.BRIDGMAN, pp. 38-41.**

**NUTRITION & CANCER, vol. 3, no. 1, 1981;
M.L.GERSHANOVICH et al., pp. 7-12.**

(73) Proprietor: **GOLD, Joseph**
**127 Edgemont Drive**
**Syracuse, NY 13214(US)**

(72) Inventor: **GOLD, Joseph**
**127 Edgemont Drive**
**Syracuse, NY 13214(US)**

(74) Representative: **Baillie, Iain Cameron**
**c/o Ladas & Parry**
**Altheimer Eck 2**
**D-80331 München (DE)**

Rank Xerox (UK) Business Services
(3.10/3.09/3.3.3)

**Description**

Many different types of chemical compounds have been used in the past to retard or inhibit various tumors in man. More than thirty compounds are approved for use in cancer therapy in various countries, but the achievement of therapeutic benefit has reached a plateau, and the search for antitumor agents continues in various directions.

In 1967, Weitzel and co-workers reported in the Zeitschrift fuer Physiologische Chemie 348, 433-442 that hydrazine acetate and sulfate inhibit in vivo the growth of ascites carcinoma and sarcoma 180 in the mouse and Walker carcinosarcoma in the rat. It is well known that the results from lower animals cannot be extrapolated in humans. Indeed, the experience at the U.S. National Institutes of Health has been that more than 200 new chemotypes having antitumor activity in animals have failed to show clinically useful anticanceractivity in humans, as shown in the following table: (Table 1 was compiled from various reports of the U.S. National Cancer Institute.)

## TABLE I

### New Chemotypes evaluated against cancer

1960-1985

**Succeeded**

Adriamycin
Cisplatin
Bleomycin
Mitomycin
Vincristine
Cyclophosphamide*
Etoposide**

\* Analog of nitrogen mustard

\*\* Analog of podophyllotoxin

**Failed**

Tripdiolide
Maytansine
Sangivamycin
Pentamethylmelamine
Taxol
Bactobolin
Alanosine
PALA
Acivicin
Methyl GAG
Menogarol
Triciribine
Disuccinimide
Flavoneacetic acid
Teroxirone
Acodazole
Benzisoquinolinedione
Didemnin B
Phyllanthoside

Ellipticine
Emetine
Indicine-N-oxide
Bouvardin
Thalicarpine
Tetrandrine
Acronycine
Tylocrebrine
Lapachol
Nitidine
Neocarzinostatin
Macromomycin ·
Largomycin
Streptimidone
Valinomycin
Piperazinedione
Fagaronine
Coralyne
Benzophenanthridine
Camptothecin
Acosamine
Acylagmatine
Allamandicin
Allamandin
Agolosamine
Anguidine
Angustmycin
Ansamycin

## TABLE I

### Failed (continued)

Aureolic acid
Baccharin
Bakkenolide
Baumycin
Bisnorditerpene
Bouvardin
Bredenine
Bruceolide
Bryogenin
Bufadienolide
Cardenolides
Catharanthus
Cephalotaxine
Chapparinone
Coformycin
Colcemid
Colchicine
Colubrinol
Coralyne
Cucurbitacin
Daphnetoxin
Datiscacin
Cecoyinine
Angustmycin
Scirpenol
Isobruceine
Nitidine
Duborimycin
Elemanolide
Elephantopin
Ellipticine
Elephantopin
Enteromycin
Eremantholides
Eriofertopin
Eudesmanolides
Eupacunin
Euparotin
Fabacein
Fagaronine
Fusarenon
Germacranolide
Glaucarubinone
Guaianolide
Helenolin
Homoerythrina
Hycanthone
Picrasin
Iridoid lactose
Isobruceine
Isocucurbitacin

## TABLE I

### Failed (continued)

Isoplumericin
Iatrophone
Lapachol
leurosidine
Leurosine
Liatrin
Masine
Maytanbutacine
Maytanbutine
Maytanprine
Maytanvaline
Miracil D
Mitrymicin
Mycophenolic acid
Neosolaneol
Nitidine
Nivalenol
Normaysine
Oxazinomycin
Peltatin
Penstemide
Phleomycin
Picrasane
Picropodophyllin
Piptocarphins
Plumericin
Porfiromycin
Pseudoguaianolides
Puromycin
Pyrazomycin
Quadrone
Quassimarin
Roridin
Samaderine
Sangivamycin
Eudesmanolide
Showdomysin
Sikkimototoxin
Simalikalactone
Simaroubolide
Stachybotrytoxin
Steganacin
Streptonigrin
Taxodione
Tenulin
Tetrandrine
Thalicarpine
Trichodermin
Undulatone
Vernolepin

## TABLE I

### Failed (continued)

Verrucarin
Vincadioline
Vindoline
Withaferin
Withanolide
Phosphonoacetic acid
Pentostatin
Deazaguanine
Tiazofurin
Ocodazole
Bisbenzimidazole
ICRF
JB-11
Dihydrotriazine benzene
  sulfonyl fluoride
Glyoxylic acid
  sulfonylhydrazone
N-Methylformamide
Caracemide
Isopropylpyrrolizine
  deriv.
Phyllanthside
Aphidicolin
Largomycin

In addition, hundreds of analogs of the new and old chemotypes have failed to show anticancer activity in man, in spite of good antitumor activity in animals. In contrast to the above, only about five new chemotype anticancer drugs have reached the market in the last 25 years. Hence early reports that hydrazine sulfate had antitumor activity in animals did not serve to predict that it might have anticancer activity in humans.

Because of this poor predictability of animal models, the National Cancer Institute of the U.S. National Institutes of Health has now abandoned the mouse model after 25 years of unproductive trial and is instituting a new in vitro program for discovering new antitumor drugs (E. Eckholm, New York Times, Dec. 23, 1986, p. C1).

A total inventory of cancer drugs approved for sale in the U.S. is set forth in Table II, and it will be seen that most of these are analogs of other drugs. Table III shows that with one exception, all of the recent New Drug Applications filed for anticancer drugs led to unapprovable ratings by the U.S. Food and Drug Administration. Table IV shows that the last new chemotype which succeeded in the clinic was discovered more than 20 years ago.

## TABLE II

Anticancer Drugs Approved in U.S. in Order of Approval by FDA.

| | | |
|---|---|---|
| leuprolide - Lupron (gonadotropin releasing hormone) | Takeda-Abbott | 4/9/85 |
| etoposide - Vepesid | BMY | 11/10/83 |
| streptozotocin - Zanosar | Upjohn | 7/7/82 |
| estramustine - Emcyt | Roche | 12/24/81 |
| daunorubicin - Cerubidine | Ives | 12/19/79 |
| cisplatin - Platinol | BMY | 12/19/78 |
| tamoxifen - Nolvadex (antiestrogen) | ICI | 12/30/77 |
| carmustine | BiCNU | 3/7/77 |
| lomustine - CEENU | BMY | 8/4/76 |
| dacarbazine - DTIC-Dome | Miles | 5/27/75 |
| doxorubicin - Adriamycin | Farmitalia | 8/7/74 |
| mitomycin C - Mutamycin | BMY | 5/28/74 |
| bleomycin - Blenoxane | BMY | 7/31/73 |
| megestrol acetate - Megace | BMY | 8/18/71 |
| floxuridine - FUDR | Roche | 12/18/70 |
| mitotane - Lysodren | BMY | 7/8/70 |
| plicamycin - Mithracin | Pfizer | 5/5/70 |
| procarbazine - Matulane | Roche | 7/22/69 |
| cytarabine - Cytosar U | Upjohn | 6/17/69 |
| testolactone - Teslac | Squibb | 6/3/69 |

| | | |
|---|---|---|
| hydroxyurea - Hydrea | Squibb | 12/7/67 |
| pipobroman - Vercyte | Abbott | 7/1/66 |
| melphalan - Alkeran | BW | 1/17/64 |
| vincristine - Oncovin | Lilly | 7/10/63 |
| uracil mustard - Uracil Mustard | Upjohn | 9/13/62 |
| 5-fluorouracil - Fluorouracil | Roche | 4/25/62 |
| dromostanolone - Drolban | Lilly | 10/26/61 |
| vinblastine - Velban | Lilly | 3/6/61 |
| cyclophosphamide - Cytoxan | BMY | 11/16/59 |
| thiotepa - Thio-Tepa | Lederle | 3/9/59 |
| chlorambucil - Leukeran | BW | 3/18/57 |
| busulfan - Myleran | BW | 6/26/54 |
| methotrexate - Methotrexate | Lederle | 12/7/53 |

A review of the FDA's New Drug Evaluation - Statistical Report (March 1986) shows that no novel anticancer drug is pending approval at the FDA.


## TABLE III

The following are the dispositions of antineoplastics filed in 1980 to 1984:

Filed in 1980  Not approvable  MDA No. 18-348 Antineoplastic
               Not approvable  MDA No. 18-529 Antineoplastic
               Not approvable  MDA No. 18-554 Antineoplastic

Filed in 1981  Not approvable  MDA No. 18-641 Antineoplastic
               Not approvable  MDA No. 18-653 Antineoplastic

Filed in 1982  Not approvable  MDA No. 50-569 Antineoplastic

Filed in 1984  Not approvable  MDA No. 50-595 Antineoplastic


Table IV lists the anticancer drugs approved in the United States. The last non-hormonal anticancer agent approved was etoposide in 1983.

The following are the years of discovery of the major anticancer drugs on the U.S. market (arbitrarily assumed to be one year before the first publication):

8

TABLE IV

| Etoposide | 1966 |
|---|---|
| Adriamycin | 1966 |
| Bleomycin | 1966 |
| Cisplatin | 1965 |
| Mitomycin C | 1965 |
| Vincristine | 1961 |
| 5-Fluorouracil | 1957 |
| Cyclophosphamide | 1957 |
| Methotrexate | 1949 |

Thus there have been no new chemotype cytotoxic cancer drugs discovered in the past twenty years. Consequently there remains an unfulfilled need for additional cancer drugs for clinical use against tumors in humans.

Up to the present time it has been generally unrecognized that a specific anticachexia agent (by virtue of its ability to interrupt those specific thermodynamic metabolic processes leading to cancer cachexia possesses antitumor potential, by virtue of a systemic thermodynamic interrelationship between tumor progression (tumor energy gain) and cancer cachexia (host energy loss); this has been taught in the scientific literature since 1974 (J. Gold, Cancer Cachexia and Gluconeogenesis, Ann. N.Y. Acad. Sci. 230, 103-110 (1974)). Thus, while it is true that any antitumor agent may have anticachexia potential, if curative, it is also true that a specific anticachexia agent may have potential for increased patient survival. However, it is not obvious, nor predictable, from the prior art that hydrazine sulfate would possess this potential.

In 1978 the present inventor was issued U.S. Patent 4,110,437 for the treatment of cancer cachexia with hydrazine sulfate. Investigations were also undertaken to ascertain whether hydrazine sulfate could retard tumor growth in humans. However, these early studies were inadequate and failed to statistically demonstrate antitumor activity.

A group at Sloan-Kettering concluded after a trial that: "The clinical observations recorded in this report fail to support a role for hydrazine sulfate as an anticancer agent. We conclude that its clinical utilization is not warranted at present and do not plan further trials." (Ochoa et al. Cancer Chemotherapy Reports Part. 1 Vol. 59 Mo. 6, Nov./Dec. 1975; pps 1151-1154).

In addition a group at the University of Virginia reported that: "Hydrazine sulfate as administered in this series failed to demonstrate any objective or subjective antitumor activity and no further trials are currently planned" (Lerner and Regelson Cancer Treatment Reports, Vol. 60, No. 7, July 1976, pps 959-966) Another later publication by Regelson et al. stated: "In conclusion we feel that hydrazine sulfate as given in this study is an inactive compound" (Cancer Chemother. Pharmacol. 3, 121-124; 1979).

Thus the prior art taught that hydrazine sulfate appeared to be inactive against primary tumor growth in man.

U.S. Patent 4,110,437 focused on the anti-cachexia effects of hydrazine sulfate. Thus, in column 2, line 17-29, it is reported that human cancer patients treated with hydrazine sulfate ceased losing weight and then gained weight. Although, maintenance of the body weight of cancer sufferers is clearly desirable, the ability of hydrazine sulfate to induce this effect does not lead one to any conclusion that it might be useful in treatment of early stages of cancer. Certainly, it provides no teaching that would lead one to a conclusion that it might be used to prepare a medicament that would actually prolong the lives of cancer sufferers if administered to them in the early Stages of cancer. Cachexia is not normally pronounced in the early stages of cancer. Thus, a teaching that a particular drug has an anti-cachexia effect does not lead one to the conclusion that it should be employed early in the treatment of cancer. Conventional chemotherapy for human cancer patientS has been directed toward the administration of cytotoxic drugs.

Against this background one skilled in the art would not have expected that a drug that was taught to have anti-cachexia effects that were applicable to late stages of cancer treatment would be useful in early stage cancer treatment. It is not apparent from what was known of the anti-cachexia effects of hydrazine sulfate that the modification of the host mechanism effected by hydrazine sulfate would lead to the possibility of prolonging the life of cancer sufferers if they were treated in the early stages of the disease.

## SUMMARY OF THE INVENTION

This invention is based on the discovery that hydrazine sulfate, when administered parenterally or orally in effective, non-toxic amounts to humans with tumors of the lung, prostate, breast, ovaries, thyroid, pancreas, lymph, cervix, gastrointestinal tract and other sites will significantly prolong survival of patients, while improving the patient's quality of life.

## DETAILED DESCRIPTION OF THE INVENTION

The dosages of hydrazine sulfate employed in the present invention can vary from 1 to 5 mg/kg daily, which is well below the LD50 and has been found to be well tolerated in the majority of patients so treated for periods of up to four years.

Preferably the regimen followed is one 60 mg capsule of hydrazine sulfate daily for the first three days, then two such capsules daily for the next three days, and then three 60 mg capsules each day thereafter.

In actual practice, patients weighing over 130 pounds do well on three or four 60 mg capsules daily, whereas patients weighing less than 100 pounds generally respond on a dosage of 30 mg administered two or three times a day. For best results blood levels of hydrazine sulfate should be determined on these patients in order to establish a most effective non-toxic dose.

I have found that treatment of patients according to the present invention at each stages of the disease is beneficial. In particular substantial benefits can be obtained by treatment with hydrazine sulfate prior to the development of cachexia.

Hydrazine sulfate therapy can advantageously be combined with other modalities for cancer treatment like chemotherapy, immunotherapy, radiation and surgery.

Hydrazine sulfate is most effective when administered usually by itself one or two hours before meals in the form of a gelatin capsule. If desired, the sulfate can be dissolved or suspended in sterile, aqueous, isotonic saline solution and given orally and parenterally. Likewise, hydrazine sulfate can be formulated with solid carriers such as talc, corn starch or stearic acid and compressed into tablets for oral administration. Such tablets can be enteric coated with shellac or cellulose acetate phthalate in a manner well known to those skilled in the pharmaceutical art.

The efficacy of hydrazine sulfate in prolonging survival in humans has now been demonstrated for the first time in a placebo-controlled, double-blind experiment with a statistically significant number of subjects.

In this experiment, to determine whether hydrazine sulfate treatment is associated with a survival benefit (R.T. Chlebowski et al., "Influence of Hydrazine Sulfate on Survival in Non-Small Cell Lung Cancer: A Randomized Placebo-Controlled Trial", to be presented at the Annual Meeting of the American Society for Clinical Oncology, May 17-19, 1987, Atlanta, Georgia), sixty-five patients with unresectable, non-small cell lung cancer and no prior chemotherapy were randomized to receive combination chemotherapy with either hydrazine sulfate or placebo addition for a period of up to four years. All received Platinol/Velban/Blenoxane (PVB) chemotherapy every 28 days, consisting of Platinol 100 mg/m$^2$; Velban 4 mg/m$^2$, days 1 and 2; and Blenoxane 10 units every 8 hours for three doses. After the initial three cycles the Blenoxane was discontinued and the Platinol dose was reduced to 50 mg/m$^2$.

Pre-chemotherapy factors including age, sex, performance status (PS), prior weight loss and disease extent were comparable in the two groups, with prechemotherapy performance status (0-1 vs 2) and prior weight loss (>10%) subsequently influencing overall survival (p<0.05). Toxicity was that expected from PVB with three patients not continuing hydrazine sulfate because of additional nausea. Survival by hydrazine sulfate or placebo group were:

| Treatment Group | Median Survival by Patient Group | |
|---|---|---|
| | All Patients | PS 0-1 Patients |
| Hydrazine Sulfate<br>Placebo | 292 days<br>173 days | 328 days*<br>209 days |

Statistical analysis of the data was by the generalized Wilcoxon (Breslau) method. Comparison of the hydrazine sulfate and placebo groups showed significance at the *P<0.01 level. All of the survival benefit of hydrazine sulfate was in the performance status 0-1 group. For the performance status (PS) 2 patients, whose condition was poor, survivals were short (median 132 days) and closely comparable whether on placebo or hydrazine sulfate. Thus, hydrazine sulfate addition, as an anticachexia agent directed primarily at correcting abnormal host metabolism and as an agent which may possess some antitumor effect, impacted on patient survival as well in patients with non-small cell lung cancer.

Several patients with tumors of the prostate, lung, breast, ovary lymph, cervix thyroid, pancreas and other tumor sites were treated with hydrazine sulfate according to the preferred regimen previously set forth.

**Claims**

1. Use of hydrazine sulfate for preparing a medicament for prolonging patients survival in an early stage cancer patient having a performance status score that is less than 2.

**Patentansprüche**

1. Verwendung von Hydrazinsulfat zur Herstellung eines Medikaments zum Verlängern des Überlebens von Patienten im frühen Krebsstadium mit einer Bewertung des Leistungsstatus von <2.

**Revendications**

1. Utilisation de sulfate d'hydrazine pour préparer un médicament destiné à prolonger la survie de cancéreux à un stade précoce ayant un score d'indice fonctionnel inférieur à 2.